## Europäisches Patentamt
## European Patent Office
## Office européen des brevets

(11) Publication number: **0 142 223**
**B1**

(12) # EUROPEAN PATENT SPECIFICATION

(45) Date of publication of patent specification: **23.11.88**

(51) Int. Cl.⁴: **A 61 M 25/00,** A 61 M 5/14

(21) Application number: **84305592.2**

(22) Date of filing: **17.08.84**

(54) Intravenous infusion assembly formed as an integral unit.

(30) Priority: **19.08.83 US 524728**

(43) Date of publication of application:
**22.05.85 Bulletin 85/21**

(45) Publication of the grant of the patent:
**23.11.88 Bulletin 88/47**

(84) Designated Contracting States:
**DE FR GB**

(56) References cited:
**EP-A-0 040 508
US-A-3 769 975
US-A-4 177 809
US-A-4 192 305**

(73) Proprietor: **Deseret Medical, Inc.
9450 South State Street
Sandy Utah 84070 (US)**

(72) Inventor: **Cartmell, Robert Lewis
2905 California Avenue
Kettering Ohio 45419 (US)**
Inventor: **Daugherty, Charles William
1726 Paintersville New Jasper Road
Xenia Ohio 45385 (US)**
Inventor: **Ireland, David Bruce
6980 Hemple Road
Dayton Ohio 45418 (US)**

(74) Representative: **Jones, Michael Raymond et al
HASELTINE LAKE & CO. Hazlitt House 28
Southampton Buildings Chancery Lane
London WC2A 1AT (GB)**

Courier Press, Leamington Spa, England.

## Description

This invention relates generally to intravenous infusion and, more particularly, to a winged catheter for intravenous infusion which is assembled from a plurality of polyurethane parts and formed into an integral unit by means of dielectric heating using radio frequency energy.

Commonly used intravenous infusion assemblies comprise needle and catheter sets wherein a needle extends within a flexible catheter having so-called "wings" attached to the catheter assembly. For insertion, one grips the wings, squeezing them between the thumb and forefinger, to assist in accurately positioning the catheter sheathed needle into a desired vein.

Once positioned, the needle is removed from the catheter leaving only the flexible catheter in place such that the vein is not likely to be ruptured as a result of minor relative movements between the infusion assembly and the vein. Varieties of combination needle/catheter sets are shown respectively in US—A—3,094,122, US—A—4,362,156, US—A—4 177 809, EP—A—0 040 508 and US—A—4 362 156.

It is important that the individual elements forming the infusion assembly be securely bonded together to prevent leakage from the assembly and detachment of the catheter. Many of the solutions passed through an intravenous infusion assembly may cause discomfort or pain if allowed to contact a patient's skin. This is particularly true with many chemotherapeutic agents utilized to treat cancer patients. Even if the solution is not toxic, bacterial contamination is a problem once an opening appears in the intravenous infusion assembly. The complications of a detached catheter within a patient's vein are potentially even graver. Accordingly, serious problems can arise if the catheter assembly is not properly manufactured.

A typical infusion assembly comprises a catheter, a winged catheter gripping member, a section of tubing and a fluid receiving hub. In the prior art, a variety of methods have been utilized to interconnect the parts making up such an assembly. The parts have been mechanically interconnected by forcing a conical wedge into the proximal end of the catheter to lock the catheter in the catheter hub. Such mechanical interconnection is often referred to as "staking" or "swedging" and can be expensive and time consuming. Furthermore, the presence of the wedge in the infusion stream creates turbulent flow which can interfere with metering the infusion liquid.

The parts have also been glued together by means of an appropriate adhesive. However, gluing requires a biocompatible adhesive and is typically messy. Gluing also can lead to inadvertent blockage of a portion of the assembly passageway particularly when small gauge catheters are utilized.

Solvent bonding has also been utilized. In solvent bonding, each of the parts is made from a material which is soluble in a particular solvent.

When the solvent is applied to the parts and the parts are intermated, dissolved surface portions of the two intermingle with one another to form a bond. Unfortunately, solvent bonding has proved to be only marginally reliable in preventing leakage. Solvent bonding also is expensive and time consuming when used for the assembly of intravenous infusion devices.

One assembly means which has been used in forming a Foley catheter unit is by welding the individual parts together using dielectric heating caused by radio frequency (RF) energy. However, radio frequency welding or bonding has not been applied in the manufacture of intravenous infusion assemblies. Problems encountered in RF bonding have centered around the frequencies and power levels utilized as well as the formation of the power applying electrodes. For example, if insufficient power is applied to the parts, the welds or bonds are not reliable. On the other hand, excessive power can lead to arcing of the radio frequency energy at the electrodes and may damage the RF power supply and/or cause the small openings of the passageways through the intravenous infusion assembly to be blocked.

Thus, it is apparent that the need exists for an integral catheter intravenous infusion assembly which will provide high reliability against leakage of possibly toxic materials onto a patient's skin as well as detachment of the catheter from the intravenous infusion assembly.

Summary of the invention

In accordance with the present invention, a unitary winged catheter assembly suitable for intravenous infusion is formed as an integral unit from a plurality of polyurethane parts of varying degrees of hardness by means of welding the parts together using dielectric heating created by radio frequency energy.

According to one aspect of the present invention, there is provided a unitary winged catheter assembly suitable for use in intravenous infusion which is formed as an integral unit from a plurality of polyurethane parts of varying degrees of hardness, the polyurethane parts being dielectrically welded together into said unitary assembly, the assembly comprising:

(a) a fluid receiving hub (102) formed from a rigid polyurethane material having a Shore D hardness within the range from 50 to 75, and having a fluid canal (116) extending from a fluid receiving first end (110) to a second end, the canal (116) having, in an intermediate region thereof, a reduced internal diameter portion (122) adjacent the region of the second end, which, with the second end region, defines a tubing stop (124) in the canal (116);

(b) a length of flexible polyurethane tubing (104) having a Shore A hardness within the range from 75 to 95, the tubing having a first end and a second end, wherein the first end of the tubing (104) and the intermediate region of the hub (102) have internal diameters substantially equal at the tubing stop (124), and wherein the first end of the tubing (104) extends into the second end region of the fluid receiving hub (102) to the tubing stop (124) and thereby forms a first joint;

(c) a gripping member (106) formed of a flexible polyurethane having a Shore A hardness within the range from 75 to 90, and having a passageway (128) extending therethrough from a first end of the gripping member to a second end thereof, the internal diameter of the gripping member passageway (128) being formed to be capable of receiving in the first end region thereof the second end of the tubing (104), and forming therewith a second joint;

(d) a pair of flexible polyurethane wings (127) having a Shore A hardness within the range from 75 to 90, positioned on the gripping member (106) and movable from a first position generally transverse to the axis of the passageway through the gripping member to a second position with the wings in facing relation to each other; and

(e) a rigid polyurethane catheter (108), having a Shore D hardness within the range from 55 to 70, having a first end and a second end, and having the second end tapered such that it is capable of being inserted into a vein, the first end of the catheter (108) being inserted into the second end region of the passageway (128) through the gripping member (106) to form a third joint, with the internal diameter of the gripping member passageway being large enough to receive in the second end region thereof said first end of the catheter (108) in order to form therewith a third joint;

wherein mating surfaces of the first, second and third joints are joined together by dielectric welding for commingling the materials of the mating surfaces, and the internal diameters of the first, second and third joints are sufficient to receive an electrode for the dielectric welding.

According to another aspect of the present invention, there is provided a method of making a unitary winged catheter assembly according to the previous aspect, the method comprising steps of:

(i) inserting the first end region of the length of tubing into the hub;

(ii) applying radio frequency energy to that zone of the hub into which the tubing is inserted, to sufficiently heat the hub zone and the tubing such that the polyurethane compounds of the two combine to weld the tubing to the hub;

(iii) inserting the second end region of the length of tubing into the first end region of the passageway in the catheter gripping member;

(iv) applying radio frequency energy to the first end region of the catheter gripping member to sufficiently heat the first end region and the inserted tubing such that the polyurethane compounds of the two combine to weld the tubing to the catheter gripping member;

(v) inserting the first end region of the catheter into the second end region of the catheter gripping member; and

(vi) applying radio frequency energy to the second end region into which the catheter is inserted, to sufficiently heat the second end region and the catheter such that the polyurethane compounds of the two combine to weld the catheter to the catheter gripping means:

wherein two or three of steps (i), (iii) and (v) are effected contemporaneously, or steps (i), (iii) and (v) are effected in any order with respect to each other; and steps (ii), (iv) and (vi) may be effected contemporaneously.

It has been noted that catheter assemblies bonded by radio frequency heating in accordance with the present invention are particularly advantageous because the assembly can be manufactured from parts made from polyurethanes having the same or different shore hardness ratings and yet these parts can be integrally bonded together such that there is intermingling of their constituent polymers and they cannot be separated.

In accordance with the present invention, a section of flexible polyurethane tubing is inserted into a polyurethane fluid receiving hub and radio frequency energy is applied to the portion of the hub into which the tubing is inserted to heat the tubing and the hub such that they melt into one another to form a reliable weld or bond. Preferably, the fluid receiving hub includes tubing stop means, for example, an internal shoulder sized to engage the end of the tubing, for defining a tubing insertion point beyond which the tubing cannot be inserted into the hub.

The opposite end of the tubing section may be inserted into a generally cylindrical central portion of a polyurethane winged catheter gripping member and radio frequency energy is again applied to weld the two together as if the two were a single part. The catheter gripping member includes a pair of wings flexibly connected to the central portion and having a relaxed condition wherein the wings extend in a generally transverse direction to the central portion, yet the wings are sufficiently flexible that they can be moved into facing engagement with one another for placement of the catheter into a selected vein. Finally, a generally cylindrical polyurethane catheter has its proximal end inserted into a canal in the generally cylindrical central portion of the catheter gripping member and is welded to it by the application of radio frequency energy with the catheter having its tapered distal end extending from the gripping member for insertion into a vein.

Such assembly and welding operations using radio frequency energy provide a winged catheter assembly which is an integral unit, i.e., the individual elements forming the infusion assembly are so bonded to one another that the polymers at the interface of two adjacent parts are intermingled.

The process for making said unitary polyurethane winged catheter assembly for intravenous infusion including a fluid receiving hub, a winged catheter gripping member, tubing for interconnecting the hub to the gripping member, and a catheter, all of which are formed from polyurethane compounds, comprises the steps of interconnecting the individual parts together and applying the appropriate frequency and energy levels of RF energy to the section of the parts receiving the tubing and the catheter such that the polyurethane compounds of the energised portions of the parts are heated to flow into one another to form an integral winged catheter assembly.

It is, therefore, an object of the present invention to provide an improved intravenous infusion assembly which is formed as an integral unit from a plurality of parts made from polyurethane compounds of different hardness with the individual parts being assembled and welded to form an integral unit by means of the application of the appropriate frequency and energy levels of radio frequency energy to interengaging portions of the assembly to thereby fuse the interengaging portions of the respective parts and firmly weld them to one another.

Advantages of the invention will be apparent from the following description and the accompanying drawings concerning particular embodiments.

Brief description of the drawings

Fig. 1 is a plan view of an intravenous infusion assembly in accordance with the present invention.

Fig. 2 is a sectional view of the fluid infusion hub of Fig. 1 taken along the line 2—2.

Fig. 3 is a sectional view of the central portion of the catheter gripping member of Fig. 1 taken along the line 3—3.

Fig. 4 is a side view of apparatus for engaging a fluid infusion hub and applying radio frequency energy to a section of that hub into which a section of tubing has been inserted.

Fig. 5 is a side view of apparatus for applying radio frequency energy to a winged catheter gripping member to thereby weld the member to polyurethane tubing inserted into one end.

Fig. 6 shows an end view of electrodes for conducting radio frequency energy to the fluid infusion hub and tubing combination.

Detailed description of the invention

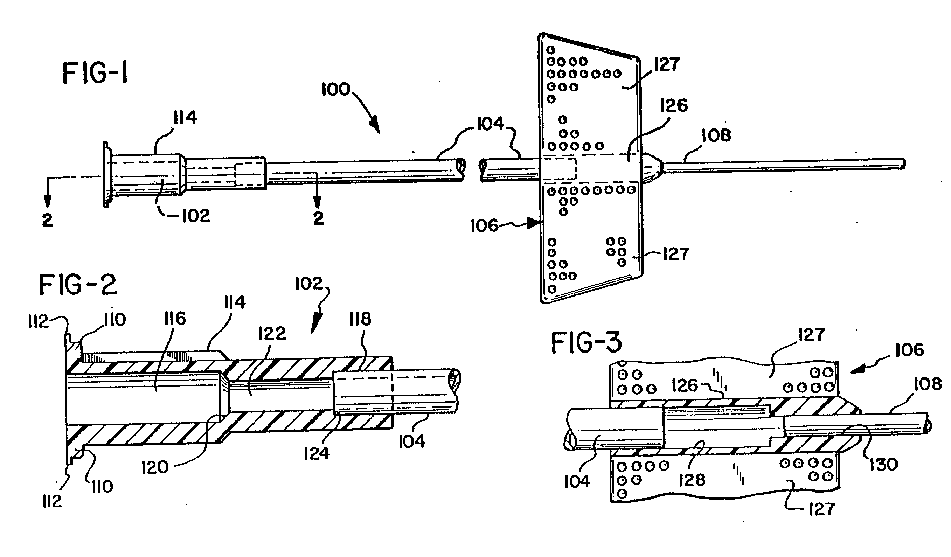

In Fig. 1, an intravenous infusion assembly 100 in accordance with the present invention comprises a fluid receiving hub 102, a section of flexible tubing 104, a winged catheter gripping member 106 and a catheter 108 all formed into an integral unit by means of radio frequency welding or bonding as will be described hereinafter.

The fluid receiving hub 102, shown in cross-section in Fig. 2, comprises a hollow, generally cylindrical, body molded from a relatively rigid polyurethane compound. Typically, the polyurethane forming the hub has a Shore Durometer D hardness of about 50 to 75 and the polyurethane forming the tubing has a Shore Durometer A of about 75 to 95.

The proximal end of the hub 102 has a collar 110 with radially and circumferentially extending lugs 112 that may be used with conventional equipment employing Luer locks for external connection to a source of infusion fluid. Reinforcing ribs 114 run approximately two-thirds of the way along the hub from the collar 110 toward the distal end of the hub.

The hub 102 comprises a primary fluid receiving cavity 116 opening to its proximal end and a tubing receiving canal 118 opening to the distal end of the hub 102. The primary fluid receiving cavity 116 and the tubing receiving canal 118 are interconnected by a diameter reducing frustoconical section 120 and a generally cylindrical interconnecting canal 122 which is of a smaller diameter than the diameter of the generally cylindrical tubing receiving canal 118. The reduced diameter canal 122 forms a tubing stop means or shoulder 124 such that when the tubing 104 is inserted into the tubing receiving canal 118, it is extended only until it engages the shoulder 124 to provide a selected or defined length of the tubing 104 extending into the hub 102.

As shown in Fig. 1, the winged catheter gripping member 106 comprises a generally cylindrical central portion 126 to which the wings 127 are flexibly connected. The gripping member is preferably formed from a relatively soft polyurethane e.g., one having a Shore Durometer A hardness of about 75 to 90 such that the wings 127, as shown in Fig. 1, can be folded upwardly into facing engagement with one another and be firmly gripped between the thumb and forefinger of a person inserting the catheter assembly into a vein of a patient. A cannula or needle (not shown) is included within the catheter assembly 100 for insertion of the catheter 108 into a vein as is well known in the art.

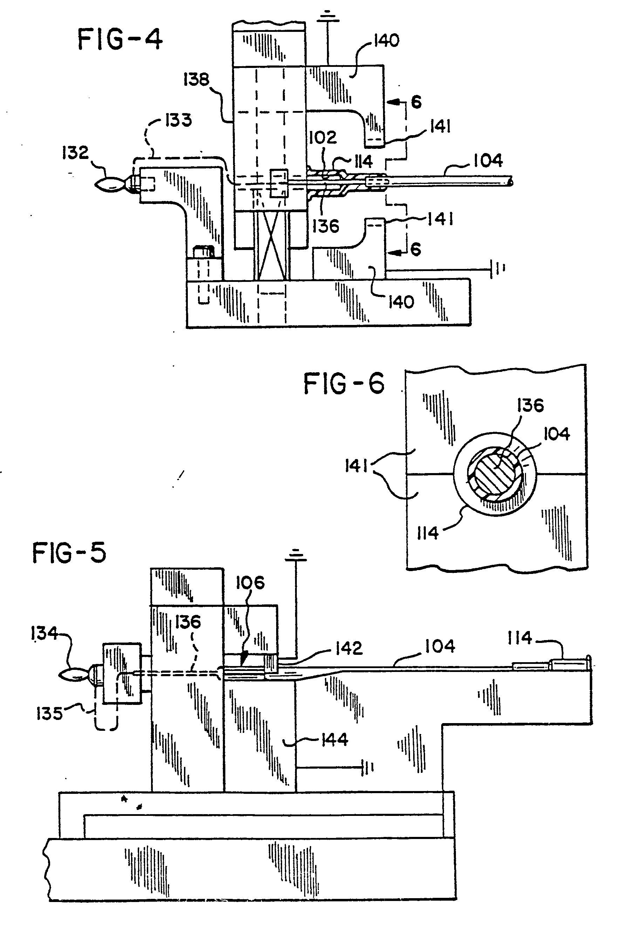

The generally cylindrical central portion 126 of the member 106, shown in cross-section in Fig. 3, includes a first opening 128 toward its proximal end sized to frictionally receive the distal end of the tubing 104. The exact insertion depth of the tubing 104 into the opening 128 is not critical. It is only necessary that the tubing 104 extend a sufficient distance into the passageway 128 such that it may be securely welded by means of dielectric heating generated by RF energy applied to the central portion 126 as will be described hereinafter.

The distal end of the central portion 126 includes a downsized opening 130 which is sized to receive the proximal end of the catheter 108. The catheter 108 may be inserted a varying depth into the opening 130 and, again, the only requirement is that a sufficient insertion depth be made such that the catheter 108 and the surrounding portion of the central section 128 forming the opening 130 may be firmly welded to one another by means of dielectric heating. A particular advantage of the present invention is that an integral catheter assembly can be formed from polyurethane elements without unduly restricting the nature of the polyurethane which forms the catheter. Thus, the catheter can be formed from a polyurethane which is hard enough to be inserted through the skin on a cannula but is sufficiently pliable as to not scratch or damage the inside wall of a vein or artery into which the catheter is inserted. Polyurethanes used for this purpose usually have a Shore Durometer D hardness of about 55 to 70.

Fig. 4 shows a jig for welding the tubing 104 to the hub 102 and Fig. 5 shows a jig for welding the tubing 104 into the central portion 126 of the winged catheter gripping member 106. RF energy from a Solidyne KH 8 RF generator from Solidyne, Inc. (not shown) is connected to terminal lugs 132 and 134 of the jigs shown in Fig. 4 and 5, respectively which in turn are connected to a metal pin 136 on which the catheter assembly is mounted as it is welded. An alternating current is delivered to the pin 136 by the generator which causes the polarity of the pin to rapidly oscillate between a highly positive and a highly negative polarity with respect to ground. Thus, an alternating electric field is set up between the pin 136 and the electrodes 140 which are grounded.

Because it is difficult to maintain uniform voltage distribution over large areas and a relatively small bond area is sufficient to form the assembly into an integral unit, electrodes 140 are designed with smaller dimension tips 141 where the field is concentrated. The portions of the catheter assembly in the alternating field between the electrode tips 141 heat in a known manner. It is not essential that there be a space between the catheter assembly 100 and the ground electrodes 140 to prevent arcing. Sharp points and edges should be avoided in the electrodes wherever possible since these are the first places breakdown occurs.

For welding the tubing 104 to the hub 102, a generally cylindrical pin 136 extends from the framework 138 of the jig shown in Fig. 4 with the generally cylindrical pin 136 being connected to the RF generator. The ground electrodes 140, an end view of which is shown in Fig. 6, are closed to engage one another and form and intimate contact from the forward portion of the hub 102 into which the tubing 104 has been inserted.

Bonding conditions will vary depending on the characteristics of the specific polyurethanes used, the electrode construction and the surroundings. The maximum voltage that can be used is limited by the voltage breakdown characteristics.

As a general rule, the frequency should be as high as possible so that the lowest voltage can be employed. However, at higher frequencies the equipment is more costly, it is difficult to deliver the power to the material as efficiently and it is more difficult to maintain uniform voltage distribution.

It has been found that RF energy at 1 to 100 mHz, preferably 25 to 70 mHz and most preferably 55 to 65 mHz is preferred for bonding the polyurethane parts of the winged catheter assembly of the present invention. However, there may be small changes in the preferred frequency as the polyurethane compositions change.

For bonding, the RF energy is connected at a power level of about 10 to 5,000 watts for a period of approximately 0.1 to 60 seconds preferably 100 to 300 watts for 2 to 11 seconds and actually at 200 watts for 4 seconds. It should be understood that the period can vary from 3 to 7 seconds depending upon the specific composition of the polyurethane compounds making up the tubing 104 and the hub 102 as well as the specific size and thickness of the hub and tubing.

The RF field is maintained for a period sufficient to melt the parts of the assembly only in the vicinity of the desired bond such that the polyurethanes forming the individual elements mingle but without losing their form or blocking the channel through the assembly. The use of the electrode pin 136 makes the upper limits of the RF power applied to weld the tubing 104 to the hub 102 somewhat less restrictive since the member 136 maintains the opening into the tubing 104 in spite of marginal excesses of heating generated by the RF energy passing through the polyurethane materials.

Fig. 5 illustrates bonding tubing 104 into the central portion of the gripping member 106. There, the ground electrode 142 is lowered to engage the central cylindrical portion 126 of the winged catheter gripping assembly 106 at the location where the tubing 104 has been inserted into the member 106. RF energy is then applied through the terminal 134 via wire 135 to pin 136 with ground being applied to the base 144 of the jig and electrode 142 such that radio frequency energy passes through the gripping member 106 and the tubing 104.

It is critical that a power level be used which forms the objective integral bond without blocking the catheter. In the case of small gauge catheters, this is especially critical and the following power levels at a frequency of 63 mHz have been found to provide a reliably secure weld of the catheter to the cylindrical member 126 without providing blockage of the small opening of the catheter.

| Catheter gauge | Power level ±15% at 5 secs |
|---|---|
| 16 | 200 watts |
| 18 | 200 watts |
| 20 | 200 watts |
| 22 | 200 watts |
| 24 | 200 watts |

While the process and product herein described constitute preferred embodiments of the invention, it is to be understood that the invention is not limited to this precise process and product and that changes may be made therein without departing from the scope of the invention which is defined in the appended claims.

**Claims**

1. A unitary winged catheter assembly suitable for use in intravenous infusion which is formed as an integral unit from a plurality of polyurethane parts of varying degrees of hardness, the polyurethane parts being dielectrically welded together into said unitary assembly, the assembly comprising:

(a) a fluid receiving hub formed from a rigid polyurethane material having a Shore D hardness within the range from 50 to 75, and having a fluid canal extending from a fluid receiving first end to a second end, the canal having, in an intermediate region thereof, a reduced internal diameter portion adjacent the region of the second end, which, with the second end region, defines a tubing stop in the canal;

(b) a length of flexible polyurethane tubing having a Shore A hardness within the range from 75 to 95, the tubing having a first end and second end, wherein the first end of the tubing and the intermediate region of the hub have internal diameters substantially equal at the tubing stop, and wherein the first end of the tubing extends into the second end region of the fluid receiving hub to the tubing stop and thereby forms a first joint;

(c) a gripping member formed of a flexible polyurethane having a Shore A hardness within the range from 75 to 90, and having a passageway extending therethrough from a first end of the gripping member to a second end thereof, the internal diameter of the gripping member passageway being formed to be capable of receiving in the first end region thereof the second end of the tubing, and forming therewith a second joint;

(d) a pair of flexible polyurethane wings, having a Shore A hardness within the range from 75 to 90, positioned on the gripping member and movable from a first position generally transverse to the axis of the passageway through the gripping member to a second position with the wings in facing relation to each other; and

(e) a rigid polyurethane catheter, having a Shore D hardness within the range from 55 to 70, having a first end and a second end, and having the second end tapered such that it is capable of being inserted into a vein, the first end of the catheter being inserted into the second end region of the passageway through the gripping member to form a third joint, with the internal diameter of the gripping member passageway being large enough to receive in the second end region thereof said first end of the catheter in order to form therewith a third joint;

wherein mating surfaces of the first, second and third joints are joined together by dielectric welding for commingling the materials of the mating surfaces, and the internal diameters of the first, second and third joints are sufficient to receive an electrode for the dielectric welding.

2. A winged catheter assembly as claimed in Claim 1, wherein the tubing stop defines a tubing insertion point beyond which the tubing cannot be inserted into the hub.

3. A winged catheter assembly as claimed in Claim 2, wherein the tubing stop comprises an internal shoulder to define the tubing insertion point sized to engage the end of the tubing as it is inserted into the hub.

4. A method of making a unitary winged catheter assembly as defined in any preceding claim, the method comprising the steps of:

(i) inserting the first end region of the length of tubing into the hub;

(ii) applying radio frequency energy to that zone of the hub into which the tubing is inserted, to sufficiently heat the hub zone and the tubing such that the polyurethane compounds of the two combine to weld the tubing to the hub;

(iii) inserting the second end region of the length of tubing into the first end region of the passageway in the catheter gripping member;

(iv) applying radio frequency energy to the first end region of the catheter gripping member to sufficiently heat the first end region and the inserted tubing such that the polyurethane compounds of the two combine to weld the tubing to the catheter gripping member;

(v) inserting the first end region of the catheter into the second end region of the catheter gripping member; and

(vi) applying radio frequency energy to the second end region into which the catheter is inserted, to sufficiently heat the second end region and the catheter such that the polyurethane compounds of the two combine to weld the catheter to the catheter gripping member;

wherein two or three of steps (i), (iii) and (v) are effected contemporaneously, or steps (i), (iii) and (v) are effected in any order with respect to each other; and steps (ii), (iv) and (Vi) may be effected contemporaneously.

5. A method according to Claim 4, wherein said radio frequency energy is applied at a frequency of 1 to 100 MHz.

6. An integral polyurethane winged catheter assembly formed in accordance with the method of Claim 4 or 5.

**Patentansprüche**

1. Für intravenöse Infusion einsetzbare Einheits-Flügel-Katheter-Anordnung (100), die als aus einer Vielzahl von Teilen aus Polyurethan unterschiedlicher Härtegrade gebildete Einheit ausgebildet ist, wobei die Polyurethanteile zu dieser Einheitsanordnung dielektrisch zusammengeschweißt sind, dadurch gekennzeichnet, daß sie

(a) eine Flüssigkeitsaufnahmenabe (102), die aus starrem Polyurethan-Material mit einer Shore-D-Härte im Bereich von Ziff 50 bis 75 gebildet ist und einen sich von einem ersten Ende zur Flüssigkeitsaufnahme zu einem zweiten Ende erstreckenden Flüssigkeitskanal (116, 120, 122, 118) aufweist, der in einem Zwischenbereich (122) von sich nahe dem Bereich des zweiten Endes ein Teilstück mit verringertem Innendurchmesser aufweist, der mit dem Bereich des zweiten Endes im Kanal (116, 120, 122, 118) einen Anschlag (124) für die Schlauchleitungsverbindung (104) bildet,

(b) ein biegsames Schlauchstück (104) aus Polyurethan-Material mit einer Shore-A-Härte im Bereich von 75 bis 95 mit einem ersten und einem zweiten Ende, wobei das erste Schlauchende und der Zwischenbereich (122) der Flüssigkeitsaufnahmenabe (102) am Schlauchleitungsanschlag (124) praktisch gleiche Innendurchmesser besitzen und sich das erste Schlauchende in den Bereich des zweiten Endes der Flüssigkeitsaufnahmenabe (102) zum Schlauchleitungsanschlag (124) erstreckt und dadurch eine erste Verbindung bildet,

(c) ein Greifglied (106), das aus biegsame Polyurethan-Material mit einer Shore-A-Härte im Bereich von 75 bis 90 gebildet ist und eine sich von einem ersten Ende von sich zu einem zweiten Ende von sich erstreckende Durchgangspassage (128, 130) aufweist, deren Innendurchmesser so ausgebildet ist, daß im Bereich ihres ersten Endes (128) das zweite Ende der Schlauchleitung (104) aufnehmbar und mit diesem eine zweite Verbindung bildbar ist,

(d) ein Paar biegsamer Flügel (127) aus Polyurethan-Material mit einer Shore-A-Härte im Bereich von 75 bis 90, die am Greifglied (106) angeordnet und aus einer im allgemeinen quer zur Achse der Durchgangspassage (128, 130) des Greifgliedes (106) verlaufenden ersten Stellung in eine zweite Stellung verbringbar sind, in welcher sie mit ihren Sichtflächen einander zugewandt sind,

(e) einen starren Katheter (108) aus Polyurethan-Material mit einer Shore-D-Härte im Bereich von 55 bis 70 mit einem ersten und einem zweiten Ende, das sich verjüngend so ausgebildet ist, daß es in eine Vene einführbar ist, wobei das erste Ende des Katheters (108) eine dritte Verbindung bildend in den Bereich des zweiten Endes (130) der Durchgangspassage (128, 130) des Greifgliedes (106) eingeführt ist, deren Innendurchmesser genügend groß ist, um zur Bildung einer dritten Verbindung mit dem Katheter (108) in ihrem zweiten Endbereich (130) das erste Ende des Katheters (108) aufzunehmen,

aufweist, wobei einander zugeordnete Oberflächen der ersten, zweiten und dritten Verbindung durch dielektrisches Verschweißen miteinander verbunden sind, indem die Materialien der einander zugeordneten Oberflächen und die Innendurchmesser der ersten, zweiten und dritten Verbindung ausreichen, eine Elektrode für das dielektrische Schweißen aufzunehmen.

2. Flügel-Katheter-Anordnung nach Anspruch 1, dadurch gekennzeichnet, daß der Schlauchleitungsanschlag (124) einen Begrenzungspunkt für das Einführen der Schlauchleitung (104) bildet, über den hinaus die Schlauchleitung (104) nicht weiter in die Flüssigkeitsaufnahmenabe (104) einführbar ist.

3. Flügel-Katheter-Anordnung nach Anspruch 2, dadurch gekennzeichnet, daß der Schlauchleitungsanschlag zur Bildung des Begrenzungspunktes für das Einführen der Schlauchleitung (104) eine Innenschulter (124) aufweist, die so bemessen ist, daß an ihr das Schlauchleitungsende zur Anlage kommt, wenn die Schlauchleitung (104) in die Flüssigkeitsaufnahmenabe (102) eingeführt ist.

4. Verfahren zu Erstellung einer Einheits-Flügel-Katheter-Anordnung (100) nach einem der vorhergehenden Ansprüche, dadurch gekennzeichnet, daß

(i) der Bereich des ersten Endes des Schlauchleitungsstückes (104) in die Flüssigkeitsaufnahmenabe (102) eingeführt wird,

(ii) die Zone der Flüssigkeitsaufnahmenabe (102), in welche die Schlauchleitung (104) eingeführt ist, Hochfrequenzenergie so ausgesetzt wird, daß die Nabenzone und die Schlauchleitung (104) ausreichend aufgeheizt werden, um die Polyurethanverbindungen beider zu solcher Verbindung miteinander zu bringen, daß die Schlauchleitung (104) sich mit der Flüssigkeitsaufnahmenabe (102) verschweißt,

(iii) der Bereich des zweiten Endes des Schlauchleitungsstückes (104) in dem Bereich des ersten Endes (128) der Durchgangspassage (128, 130) im Greifglied (106) des Katheters (108) eingeführt wird,

(iv) der Bereich des ersten Endes des Kathetergreifgliedes (106) Hochfrequenzenergie so ausgesetzt wird, daß dieser erste Endbereich und die in ihn eingeführte Schlauchleitung (104) ausreichend aufgeheizt werden, um die Polyurethanverbindungen beider zu solcher Verbindung miteinander zu bringen, daß die Schlauchleitung (104) sich mit dem Greifglied (106) des Katheters (108) verschweißt,

(v) der Bereich des ersten Endes des Katheters (108) in den Bereich des zweiten Endes des Katheter-Greifgliedes (106) eingeführt wird und

(vi) der Bereich des zweiten Endes des Kathetergreifgliedes (106), in den der Katheter (108) eingeführt ist, Hochfrequenzenergie so ausgesetzt wird, daß dieser zweite Endbereich des Greifgliedes (106) und der Katheter (108) ausreichend auf aufgeheizt werden, um die Polyurethanverbindungen beider zu solcher Verbindung miteinander zu bringen, daß sich der Katheter (108) mit dem Kathetergreifglied (106) verschweißt,

wobei zwei oder drei der Verfahrensschritte (i), (iii) und (v) gleichzeitig oder in beliebiger Folge zueinander und die Verfahrensschritte (ii), (iv) und (vi) gleichzeitig durchführbar sind.

5. Verfahren nach Anspruch 4, dadurch gekennzeichnet, daß Hochfrequenzenergie mit einer Frequenz von 1 bis 100 MHz eingesetzt wird.

6. Einstückige Flügel-Katheter-Anordnung (100) aus Polyurethan-Material, dadurch gekennzeichnet, daß sie nach dem Verfahren gemäß Anspruch 4 oder 5 erstellt ist.

**Revendications**

1. Un ensemble de cathéter unitaire à oreilles approprié pour l'utilisation en infusion intra-veineusé, réalisé en unité monobloc à partir d'une pluralité de pièces en polyuréthane de différents degrés de dureté, les pièces en polyuréthane étant soudées diélectriquement ensemble pour former le dit ensemble unitaire, l'ensemble comprenant:

(a) un moyeu de réception de liquide formé à partir d'un matériau polyuréthane rigide ayant une dureté Shore D se situant dans la gamme de 50 à 75, et comportant un canal de passage de liquide s'étendant d'une première extrémité de réception de liquide à une deuxième extrémité, le canal, dans une zone intermédiaire de celui-ci, présentant une partie de diamètre intérieure réduit adjacente à la zone de la deuxième extrémité, cette partie de diamètre réduit, avec la zone de deuxième extrémité, définissant une butée d'arrêt de tube dans le canal;

(b) une longueur de tube en polyuréthane flexible ayant une dureté Shore A se situant dans la gamme de 75 à 95, le tube comportant une première extrémité et une deuxième extrémité, la première extrémité du tube et la zone intermédiaire du moyeu ayant des diamètres intérieurs rigoureusement égaux à la butée d'arrêt de tube, la première extrémité du tube s'étendant dans la zone de deuxième extrémité du moyeu de réception de liquide jusqu'à la butée d'arrêt de tube, et formant ainsi un premier raccord;

(c) un élément de prise ou de préhension réalisé en polyuréthane flexible ayant une dureté Shore A se situant dans la gamme de 75 à 90, et comportant une voie de passage s'étendant de bout en bout depuis une première extrémité de l'élément de préhension jusqu'à une deuxième extrémité de cet élément, le diamètre intérieur de la voie de passage de l'élément de préhension étant prévu de manière à pouvoir recevoir dans la première zone d'extrémité de cette voie de passage la deuxième extrémité du tube, en formant avec celle-ci un deuxième raccord;

(d) une paire d'oreilles en polyuréthane flexible, ayant une dureté Shore A se situant dans la gamme de 75 à 90, positionnées sur l'élément de préhension et mobiles depuis une première position généralement transversale par rapport à l'axe de la voie de passage dans l'élément de préhension, jusqu'à une deuxième position avec les oreilles se faisant face l'une l'autre; et

(e) un cathéter en polyuréthane rigide, ayant une dureté Shore D se situant dans la gamme de 55 à 70, comportant une première extrémité et une deuxième extrémité, la deuxième extrémité étant profilée tronconique de manière à pouvoir être introduite dans une veine, la première extrémité du cathéter étant introduite dans la deuxième zone d'extrémité de la voie de passage dans l'élément de préhension de façon à former un troisième raccord, le diamètre intérieur de la voie de passage de l'élément de préhension étant suffisamment grand pour recevoir dans la deuxième zone d'extrémité de cette voie de passage la dite première extrémité du cathéter pour la formation du troisième raccord,

les surfaces concordantes des premier, deuxième et troisième raccords étant jointées ensemble par soudure diélectrique pour le mélange intime des matériaux des surfaces concordantes, et les diamètres intérieures des premier, deuxième et troisième raccords étant suffisants pour recevoir une électrode pour la soudure diélectrique.

2. Un ensemble de cathéter à oreilles selon la revendication 1, caractérisé en ce que la butée d'arrêt de tube définit un point d'introduction de tube au-delà duquel le tube ne peut pas être inséré dans le moyeu.

3. Un ensemble de cathéter à oreilles selon la revendication 2, caractérisé en ce que la butée d'arrêt de tube comprend un épaulement intérieur pour définir le point d'introduction de tube, cet épaulement étant dimensionné pour s'engager avec l'extrémité du tube lorsque ce dernier est introduit dans le moyeu.

4. Une méthode de fabrication d'un ensemble de cathéter unitaire à oreilles tel que défini dans l'une quelconque des revendications precédentes, la méthode comprenant les opérations suivantes:

(i) insertion de la première zone d'extrémité de la longueur de tube dans le moyeu;

(ii) application d'énergie de radio-fréquence sur la zone du moyeu dans laquelle le tube est introduit, de manière à chauffer suffisamment la zone de moyeu et le tube pour que les composés polyuréthane du moyeu et du tube se combinent de façon à souder le tube au moyeu;

(iii) insertion de la deuxième zone d'extrémité de la longueur de tube dans la première zone d'extrémité de la voie de passage dans l'élément de préhension du cathéter;

(iv) application d'énergie de radio-fréquence sur la première zone d'extrémité de l'élément de préhension du cathéter, de manière à chauffer suffisamment la première zone d'extrémité et le tube introduit pour que les composés polyuréthane de cette zone d'extrémité et du tube se combinent de façon à souder le tube à l'élément de préhension du cathéter;

(v) insertion de la première zone d'extrémité du cathéter dans la deuxième zone d'extrémité de l'élément de préhension du cathéter; et

(vi) application d'énergie de radio-fréquence sur la deuxième zone d'extrémité dans laquelle le cathéter est introduit, de manière à chauffer suffisamment la deuxième zone d'extrémité et le cathéter pour que les composés polyuréthane de cette deuxième zone d'extrémité et du cathéter se combinent de façon à souder le cathéter à l'élément de préhension du cathéter,

deux des opérations (i), (iii) et (v) ou les trois étant effectuées simultanément, ou bien les opérations (i), (iii) et (v) étant effectuées en tout ordre quelconque l'une par rapport à l'autre, les opérations (ii), (iv) et (vi) pouvant être effectuées simultanément.

5. Une méthode selon la revendication 4, caractérisé en ce que la dite énergie de radio-fréquence est appliquée à une fréquence de 1 à 100 mHz.

6. Un ensemble monobloc de cathéter à oreilles en polyuréthane, réalisé selon la méthode de la revendication 4 ou 5.

FIG-1

FIG-2

FIG-3

EP 0 142 223 B1

FIG-4

FIG-6

FIG-5